(19) Europäisches Patentamt
European Patent Office
Office européen des brevets

(11) **EP 2 505 662 B1**

(12) **EUROPEAN PATENT SPECIFICATION**

(45) Date of publication and mention of the grant of the patent:
**27.01.2016 Bulletin 2016/04**

(51) Int Cl.:
*C12Q 1/68* (2006.01)  *C12M 1/00* (2006.01)
*C12M 1/34* (2006.01)  *G01N 33/50* (2006.01)
*C12N 15/09* (2006.01)  *G01N 33/68* (2006.01)

(21) Application number: **10833015.0**

(22) Date of filing: **26.10.2010**

(86) International application number:
**PCT/JP2010/068909**

(87) International publication number:
**WO 2011/065168 (03.06.2011 Gazette 2011/22)**

(54) **METHOD AND APPARATUS FOR PREDICTION OF PHARMACOLOGICAL EFFICACY OF HUMANIZED ANTI-TNF  ANTIBODY DRUG ON RHEUMATOID ARTHRITIS**

VERFAHREN UND VORRICHTUNG ZUR VORHERSAGE DER PHARMAKOLOGISCHEN WIRKSAMKEIT EINES WIRKSTOFFS AUF EINEM  ANTI-TNF-ANTIKÖRPER ZUR BEHANDLUNG VON RHEUMATOIDER ARTHRITIS

PROCÉDÉ ET APPAREIL DE PRÉDICTION DE L'EFFICACITÉ PHARMACOLOGIQUE D'UN MÉDICAMENT À BASE D'ANTICORPS ANTI-TNF  HUMANISÉ SUR LA POLYARTHRITE RHUMATOÏDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **24.11.2009 JP 2009266539**

(43) Date of publication of application:
**03.10.2012 Bulletin 2012/40**

(73) Proprietor: **KayteeBio Co. & Ltd.**
**Chiba 273-0864 (JP)**

(72) Inventor: **TSUZAKA, Kensei**
**Funabashi-shi,**
**Chiba 2730864 (JP)**

(74) Representative: **Graf von Stosch, Andreas et al**
**Graf von Stosch**
**Patentanwaltsgesellschaft mbH**
**Prinzregentenstrasse 22**
**80538 München (DE)**

(56) References cited:
**EP-A1- 2 343 372     WO-A1-02/31163**

- **KENSEI TSUZAKA ET AL: "Toyomae Kecchu ADAMTS5 Hatsugenryo o Moto ni shita RA ni Taisuru Infliximab Yukosei Yosoku [=ADAMTS5 (a disintegrin and metalloproteinase with thrombospondin motifs 5)]", THE 53RD ANNUAL GENERAL ASSEMBLY AND SCIENTIFIC MEETING OF JAPAN COLLEGE OF RHEUMATOLOGY THE 18TH INTERNATIONAL RHEUMATOLOGY SYMPOSIUM, JAPAN COLLEGE OF RHEUMATOLOGY, JAPAN, vol. 53RD-18TH, 1 March 2009 (2009-03-01), page 274, XP008155787,**
- **KENSEI TSUZAKA ET AL.: 'Toyomae Kecchu ADAMTS5 Hatsugenryo o Moto ni shita RA ni Taisuru Infliximab Yukosei Yosoku' JAPAN COLLEGE OF RHEUMATOLOGY SOKAI GAKUJUTSU SHUKAI KOKUSAI RHEUMATOLOGY SYMPOSIUM PROGRAM SHOROKUSHU vol. 53RD-18T, March 2009, page 274, XP008155787**

**Description**

Technical Field

The present invention relates to a method for predicting pharmacological efficacy of a human anti-TNFα antibody drug against rheumatoid arthritis based on an index that is the level of at least one of ADAMTS4 and ADAMTS5 in a sample derived from a subject.

Background Art

In recent years, biologicals targeting TNFα (tumor necrosis factor α) have been used for remission of rheumatoid arthritis, and their efficacy has been recognized. Adalimumab (ADA) is the biological containing a fully human anti-TNFα monoclonal antibody and targets TNFα. It has already been found that adalimumab suppresses the activity of rheumatoid arthritis (RA) from a clinical index referred to as DAS (Disease activity score) 28. Also, some studies have reported that adalimumab not only suppresses the activity of rheumatoid arthritis, but also suppresses bone destruction associated with rheumatoid arthritis.

Regardless of such remarkable efficacy, there are some cases where rheumatoid arthritis does not respond to adalimumab. Furthermore, adalimumab is prone to cause, as adverse side effects, infectious diseases such as Pneumocystis pneumonia and pulmonary tuberculosis. Thus, predicting adalimumab efficacy prior to administration thereof allows us to administer adalimumab to patients on which adalimumab reliably takes effects, with preventing unwanted side effects.

There have been previous reports about attempts to identify factors for efficacy prediction of biologicals such as adalimumab (see NPL 1). NPL 1 reports that combinations of 439 genes are correlated with being inefficacious against adalimumab on the basis of the finding obtained as follows. Specifically, after administration of adalimumab for 12 weeks, differences of gene expression profiles between adalimumab-efficacious RA patients and adalimumab-inefficacious RA patients were obtained from analyses of their synovial membrane samples using DNA microarrays and algorithms.

However, this method is the retrospective study and does not quantify the efficacy of adalimumab. Thus, at present, reliable predictive factors of the efficacy of adalimumab have not been identified yet in the prospective study.

It is known that ADAMTS (a disintegrin and metalloproteinase with thrombospondin motifs) 4 and ADAMTS 5 are aggrecanases that belong to the ADAMTS family and are involved in cartilage destruction. In particular, some studies reported that deletion of ADAMTSS gene results in preventing cartilage destruction in the murine model of degenerative arthritis.

Despite that, it has not been known whether or not the expression level of at least one of ADAMTS4 and ADAMTS5 associates with the efficacy of a human anti-TNFα antibody drug against rheumatoid arthritis.

Citation List

Non-Patent Literature

NPL 1: Badot V, et al. Arthritis Research Therapy 11: R57, 2009

EP 2 343 372 teaches a method for predicting pharmacological efficacy of a human anti-TNFα antibody drug against rheumatoid arthritis, the method comprising measuring the amount of ADAMTS5 in a sample derived from a subject and determining whether or not the human anti-TNFα antibody drug is efficacious against rheumatoid arthritis of the subject, based on the level of ADAMTS5 serving as an index. The publication by Kensei Tsuzaka et al. (The 54rd annual general assembly and scientific meeting of Japan College of Rheumatology the 18th International Rheumatology Symposium, Japan College of Rheumatology, Japan, vol. 53rd-18th, 1 March 2009, page 274) teaches efficacious prediction of infliximab against rheumatoid arthritis on the basis of the blood expression level of ADAMTS5 before administration.

Summary of Invention

Technical Problem

The present invention aims to solve the above-described problems and achieve the following objects. Specifically, the object of the present invention is to provide a method for predicting pharmacological efficacy of a human anti-TNFα antibody drug against rheumatoid arthritis, the method being able to predict the efficacy of a human anti-TNFα antibody drug against rheumatoid arthritis with high reliability and in a simple manner as well as being able to predict the activity of rheumatoid arthritis after administration of the human anti-TNFα antibody drug, resulting in that the human anti-TNFα antibody drug can be administered to patients on which the human anti-TNFα antibody drug reliably takes effects with preventing unwanted side effects.

Solution to Problem

The present inventors conducted extensive studies to solve the above problems and have found that the higher the level of at least one of ADAMTS4 and ADAMTS5 in a peripheral blood sample collected from a patient with rheumatoid arthritis before administration of adalimumab, the more efficacious adalimumab against rheumatoid arthritis. No prospective study has shown that the efficacy of adalimumab could be predicted by measuring the level of at least one of ADAMTS4 and ADAMTS5 in a blood sample collected before administration of adalimumab. Thus, this is the new finding obtained by the present inventors.

The "retrospective study" refers to a study that analyzes previous data and present data, while the "prospective study" refers to a study that observes a phenomenon occurring in the future. The prospective study is superior to the retrospective study since the prospective study can yield more reliable results. This is because the retrospective study is performed on the already known matter so that it may easily contain researchers' bias, while the prospective study is performed on unknown matter and thus is free from researchers' bias.

The present invention is based on the above finding obtained by the present inventors. Means for solving the above existing problems are as follows.

<1> A method for predicting pharmacological efficacy of a human anti-TNFα antibody drug against rheumatoid arthritis, according to claim 1.

<2> The method according to <1>, wherein the human anti-TNFα antibody drug is adalimumab (ADA).

<3> The method according to <1> or <2>, wherein the expression level of the at least one of ADAMTS4 mRNA and ADAMTS5 mRNA is measured by real-time PCR method.

Non-claimed embodiments are:

<a> An apparatus for predicting pharmacological efficacy of a human anti-TNFα antibody drug against rheumatoid arthritis, the apparatus including:

a measuring unit configured to measure a level of at least one of ADAMTS4 and ADAMTS5 in a sample derived from a subject, and
a determining unit configured to determine whether or not the human anti-TNFα antibody drug is efficacious against rheumatoid arthritis of the subject, based on the level of the at least one of ADAMTS4 and ADAMTS5 serving as an index.

<b> The apparatus according to <a>, wherein the human anti-TNFα antibody drug is adalimumab (ADA).

<c> The apparatus according to <a> or <b>, wherein the level of the at least one of ADAMTS4 and ADAMTS5 in the sample derived from the subject is an expression level of at least one of ADAMTS4 mRNA and ADAMTS5 mRNA.

<d> The apparatus according to <c>, wherein the expression level of the at least one of ADAMTS4 mRNA and ADAMTS5 mRNA is measured by real-time PCR method.

Advantageous Effects of Invention

The present invention can provide a method for predicting pharmacological efficacy of a human anti-TNFα antibody drug against rheumatoid arthritis, the method being able to predict the efficacy of a human anti-TNFα antibody drug against rheumatoid arthritis with high reliability and in a simple manner as well as being able to predict the activity of rheumatoid arthritis after administration of a human anti-TNFα antibody drug, resulting in that the human anti-TNFα antibody drug can be administered to patients on which the human anti-TNFα antibody drug reliably takes effects with preventing unwanted side effects. These methods can solve the above existing problems and achieve the above-described objects.

Brief Description of Drawings

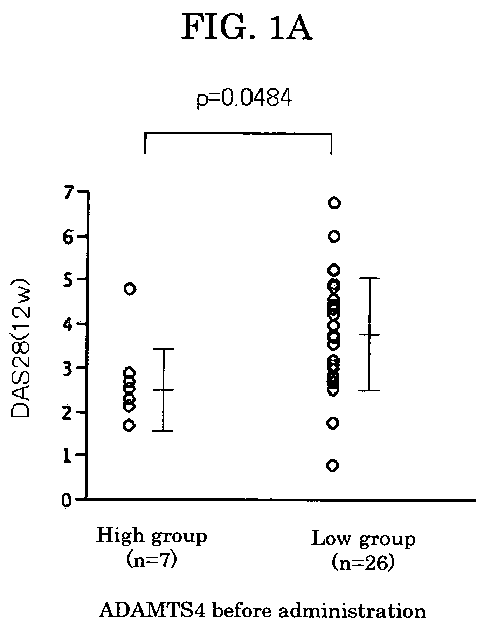

Fig. 1A is a graph showing DAS28 (12w) at 12 weeks after administration of adalimumab in the Low group where the expression level of ADAMTS4 mRNA (ADAMTS4/β-actin) before administration of adalimumab is lower than $0.3 \times 10^{-4}$, and the High group where the expression level of ADAMTS4 mRNA (ADAMTS4/β-actin) before administration of adalimumab is equal to or higher than $0.3 \times 10^{-4}$.

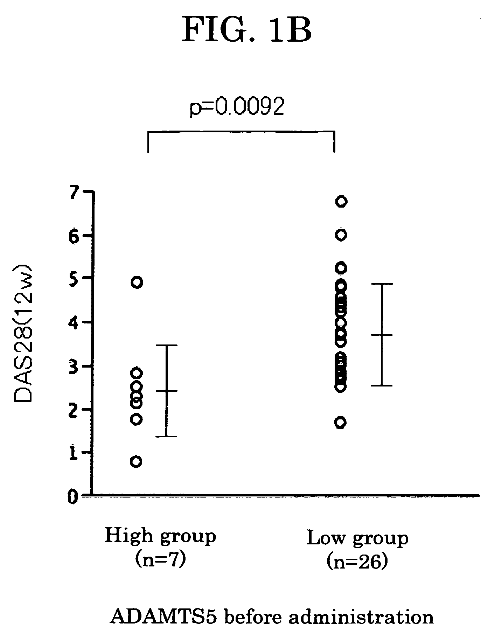

Fig. 1B is a graph showing DAS28 (12w) at 12 weeks after administration of adalimumab in the Low group where the expression level of ADAMTS5 mRNA (ADAMTS5/β-actin) before administration of adalimumab is lower than $4.0 \times 10^{-4}$, and the High group where the expression level of ADAMTS5 mRNA (ADAMTS5/β-actin) before administration of adalimumab is equal to or higher than $4.0 \times 10^{-4}$.

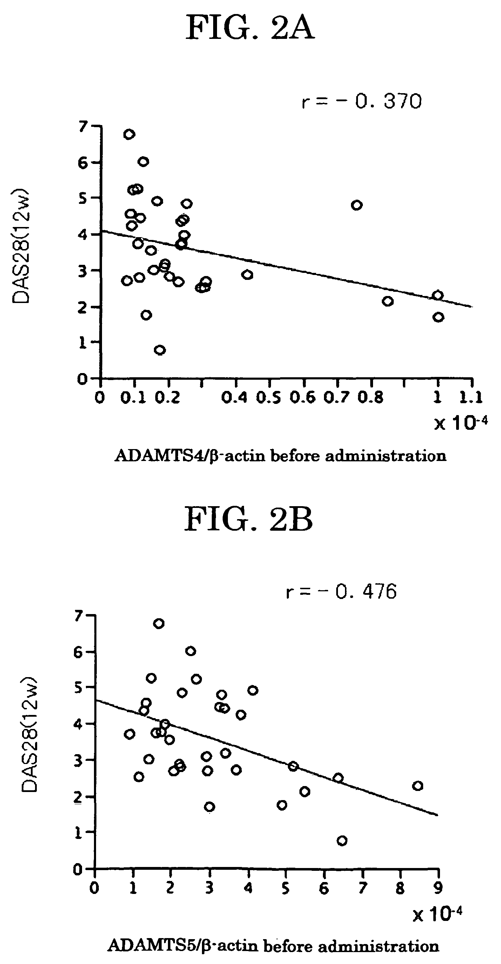

Fig. 2A is a graph showing correlation between the expression level of ADAMTS4 mRNA(ADAMTS4/β-actin) before administration of adalimumab and the change of DAS28 (12w) at 12 weeks after administration of adalimumab.

Fig. 2B is a graph showing correlation between the expression level of ADAMTS5 mRNA (ADAMTS5/β-actin) before administration of adalimumab and the change of DAS28 (12w) at 12 weeks after administration of adalimumab.

Description of Embodiments

(A method for predicting pharmacological efficacy of a human anti-TNFα antibody drug against rheumatoid arthritis)

A method of the present invention for predicting pharmacological efficacy of a human anti-TNFα antibody drug against rheumatoid arthritis according to claim 1 includes at least measuring the level of at least one of ADAMTS4 and ADAMTS5 in a sample derived from a subject (measuring step) and determining whether or not the human anti-TNFα antibody drug is efficacious against rheumatoid arthritis of the subject, based on the level of the at least one of ADAMTS4 and ADAMTS5 serving as an index (determining step); and, if necessary, further includes other steps.

A (non-claimed) apparatus for predicting pharmacological efficacy of a human anti-TNFα antibody drug against rheumatoid arthritis includes at least a unit configured to measure the level of at least one of ADAMTS4 and ADAMTS5 in a sample derived from a subject (measuring unit) and a unit configured to determine whether or not the human anti-TNFα antibody drug is efficacious against rheumatoid arthritis of the subject, based on the level of the at least one of ADAMTS4 and ADAMTS5 serving as an index (determining unit); and, if necessary, further includes other units.

<A measuring step and a measuring unit>

The measuring step of the claimed method is a step of measuring the level of at least one of ADAMTS4 and ADAMTS5 in a sample derived from a subject.

The measuring unit of the non-claimed apparatus is a unit configured to measure the level of at least one of ADAMTS4 and ADAMTS5 in a sample derived from a subject.

-A sample derived from a subject-

The sample derived from a subject is not particularly limited and may be appropriately selected depending on the intended purpose. Examples thereof include peripheral blood, a synovial membrane and synovial fluid. In particular, peripheral blood is preferred because it is easy to sample.

The subject is not particularly limited and may be appropriately selected depending on the intended purpose. Examples thereof include human.

-ADAMTS4-

The ADAMTS (a disintegrin and metalloproteinase with thrombospondin motifs) 4 is an aggrecanase that belongs to the ADAMTS family and is involved in cartilage destruction.

The nucleotide sequence of ADAMTS4 genes is heretofore known, for example that of human. The nucleotide sequence is easily available from public databases such as GenBank (NCBI). For example, the nucleotide sequence of human ADAMTS4 gene is available under NCBI accession number NM_005099.

-ADAMTS5-

The ADAMTS (a disintegrin and metalloproteinase with thrombospondin motifs) 5 is an aggrecanase that belongs to the ADAMTS family and is involved in cartilage destruction.

The nucleotide sequence of ADAMTS5 genes is heretofore known, for example that of human. The nucleotide sequence is easily available from public databases such as GenBank (NCBI). For example, the nucleotide sequence of human ADAMTS5 gene is available under NCBI accession number NM_007038.

-Measurement of a level-

The method for measuring the level of at least one of ADAMTS4 and ADAMTS5 in the sample derived from a subject is not particularly limited and may be appropriately selected depending on the intended purpose. Examples thereof include a method for measuring the mRNA expression level and a method for measuring the protein expression level.

The method for measuring the mRNA expression level is not particularly limited and may be appropriately selected depending on the intended purpose. Examples thereof include PCR method, real-time PCR method, DNA array method, and Northern blotting method.

The non-claimed apparatus for measuring the mRNA expression level is not particularly limited and may be appropriately selected depending on the intended purpose. Examples thereof include PCR apparatus, real-time PCR apparatus, DNA array apparatus, and Northern blotting apparatus. Each of the above apparatuses can be suitably used as the measuring unit.

The real-time PCR method is not particularly limited and may be appropriately selected depending on the intended purpose. Examples thereof include a method including preparing a calibration curve and quantifying a sample based on the calibration curve (calibration curve method).

The template DNA control used for the calibration curve method is not particularly limited and may be appropriately selected depending on the intended purpose. Examples thereof include the total cDNA obtained and purified from healthy human, a plasmid into which ADAMTS4 cDNA has been incorporated, and a plasmid into which ADAMTS5 cDNA has been incorporated.

The primers used in the real-time PCR method are not particularly limited, so long as at least one of ADAMTS4 and ADAMTS5 can be amplified, and may be appropriately selected depending on the intended purpose.

The endogenous control used in the real-time PCR method is not particularly limited and may be appropriately selected depending on the intended purpose. Examples thereof include β-actin and GAPDH (glyceraldehyde-3-phosphate dehydrogenase).

<A determining step and a determining unit>

The determining step of the claimed method is a step of determining whether or not the human anti-TNFα antibody drug is efficacious against rheumatoid arthritis of the subject, based on the level of at least one of ADAMTS4 and ADAMTS5 serving as an index.

The determining unit of the non-claimed apparatus is a unit configured to determine whether or not the human anti-TNFα antibody drug is efficacious against rheumatoid arthritis of the subject, based on the level of at least one of ADAMTS4 and ADAMTS5 serving as an index.

-A human anti-TNFα antibody drug-

The human anti-TNFα (tumor necrosis factor α) antibody drug is not particularly limited and may be appropriately selected depending on the intended purpose. For example, adalimumab (ADA) is preferred.

-Index-

As the index, the level of at least one of ADAMTS4 and ADAMTS5 is used. Either the level of ADAMTS4 or the level of ADAMTS5 may be used as the index, or both of them may be used in combination as the index.

-Determination-

The method for determining whether or not the human anti-TNFα antibody drug is efficacious against rheumatoid arthritis is not particularly limited and may be appropriately selected depending on the intended purpose. For example, when the level of at least one of ADAMTS4 and ADAMTS5 in a patient with rheumatoid arthritis is more than that in other patients with rheumatoid arthritis, a human anti-TNFα antibody drug can be determined as being efficacious against rheumatoid arthritis of the patient.

According to claim 1, in the case where the level of ADAMTS4 is used as the index (details will be described in the below Example 1), when the expression level of ADAMTS4 mRNA (ADAMTS4/β-actin) is $0.3 \times 10^{-4}$ or higher, a human anti-TNFα antibody drug can be determined as being efficacious against rheumatoid arthritis.

Meanwhile, in the case where the level of ADAMTS5 is used as the index according to claim 1 (details will be described in the below Example 1), when the expression level of ADAMTS5 mRNA (ADAMTS5/β-actin) is $4.0 \times 10^{-4}$ or higher, a human anti-TNFα antibody drug can be determined as being efficacious against rheumatoid arthritis.

The non-claimed device for the determination is not particularly limited and may be appropriately selected depending on the intended purpose. Examples thereof include an electronic calculator (a computer). The above device can be suitably used as the determining unit.

The determination also enables DAS28 to be predicted after administration of a human anti-TNFα antibody drug.

The "DAS28" is an abbreviation of Disease Activity Score which is established by the European League Against Rheumatism (EULAR) in order to score the activity of rheumatoid arthritis and described in Fransen J, et al. Clin Exp Rheumatol 2005; 23 (Suppl. 39): S93-S99.

The method for predicting DAS28 after administration of a human anti-TNFα antibody drug is not particularly limited and may be appropriately selected depending on the intended purpose. One exemplary method for predicting DAS28 after administration of a human anti-TNFα antibody drug includes: measuring the expression level of at least one of ADAMTS4 mRNA and ADAMTS5 mRNA (at least one of ADAMTS4/β-actin and ADAMTS5/β-actin) in peripheral blood of a patient with rheumatoid arthritis; and fitting the measured expression level to the graph referred to in the below Example 1 (see Figs. 2A and 2B).

<Other steps and other units>

The other steps of the claimed method are not particularly limited and may be appropriately selected depending on the intended purpose. Examples thereof include a step of performing the determination considering at least one of ADAMTS4 and ADAMTS5 in combination with other genes.

The other units of the non-claimed apparatus are not particularly limited and may be appropriately selected depending on the intended purpose. Examples thereof include a unit configured to perform the determination considering at least one of ADAMTS4 and ADAMTS5 in combination with other genes.

-Other genes-

The other genes are not particularly limited and may be appropriately selected depending on the intended purpose. Examples thereof include genes encoding aggrecanases other than ADAMTS4 and ADAMTS5, and genes expressing specifically in subjects with rheumatoid arthritis.

Examples

The present invention will next be described in detail by way of Examples, which should not be construed as limiting the present invention thereto.

(Example 1: Prediction of efficacy of adalimumab (ADA) against rheumatoid arthritis based on the level of ADAMTS4 or ADAMTS5 serving as an index)

Thirty-three patients with rheumatoid arthritis were enrolled in this study. They visited the Division of Rheumatology, Department of Internal Medicine, Saitama Medical Center, Saitama Medical University from June in 2008 to June in 2009, and received adalimumab.

<Measuring step>

-Measurement of the expression level of ADAMTS4 mRNA in peripheral blood-

Peripheral blood (2.5 mL) was sampled from each of the patients with rheumatoid arthritis before administration of adalimumab, and then placed in a PAXGENE BLOOD RNA TUBE (registered trademark, product of Becton, Dickinson and Company Japan), followed by isolation of the total RNA using a PAXGENE BLOOD RNA KIT (registered trademark, product of PreAnalytiX GmbH). The total RNA was converted to the total cDNA using reverse transcriptase. Using the total cDNA as a template DNA, the expression level of ADAMTS4 mRNA was analyzed by real-time PCR method using TAQMAN (registered trademark) GENE EXPRESSION ASSAY (product of Applied Biosystems). The primers and probes used were those of a primer-probe set (TAQMAN GENE (registered trademark) EXPRESSION ASSAY (Hs00192708_m1, product of Applied Biosystems).

The level of ADAMTS4 mRNA obtained above was quantified as the relative value to the level of mRNA of β-actin (endogenous control).

The primers and probes of β-actin were those of a primer-probe set (PRE-DEVELOPED TAQMAN (registered trademark) ASSAY REAGENTS (Human ACTB, NM_001101, product of Applied Biosystems)).

-Measurement of the expression level of ADAMTS5 mRNA in peripheral blood-

Peripheral blood (2.5 mL) was sampled from each of the patients with rheumatoid arthritis before administration of adalimumab, and then placed in a PAXGENE BLOOD RNA TUBE (registered trademark, product of Becton, Dickinson and Company Japan), followed by isolation of the total RNA using a PAXGENE BLOOD RNA KIT (registered trademark, product of PreAnalytiX GmbH). The total RNA was converted to the total cDNA using reverse transcriptase. Using the total cDNA as a template DNA, the expression level of ADAMTS5 mRNA was analyzed by real-time PCR method using TAQMAN (registered trademark) GENE EXPRESSION ASSAY (product of Applied Biosystems). The primers and probes used were those of a primer-probe set (TAQMAN GENE (registered trademark) EXPRESSION ASSAY (00199841_m1, product of Applied Biosystems).

The level of ADAMTS5 mRNA obtained above was quantified as the relative value to the level of mRNA of β-actin (endogenous control).

The primers and probes of β-actin were those of a primer-probe set (Pre-Developed TAQMAN (registered trademark) ASSAY REAGENTS (Human ACTB, NM_001101, product of Applied Biosystems)).

<Determining step>

The activity of each rheumatoid arthritis patient was determined based on DAS28 before administration of adalimumab and on DAS28 at 12 weeks after administration of adalimumab (DAS28 (0w) and DAS28 (12w)).

Regarding whether adalimumab was efficacious or not, the patients were determined as "good responder," "moderate responder" or "non responder" by evaluating their DAS28 (12w) according to the EULAR response criteria.

The EULAR response criteria are established by European League Against Rheumatism (EULAR) and described in Fransen J, et al. Clin Exp Rheumatol 2005; 23 (Suppl. 39): S93-S99.

Regarding the efficacy of adalimumab at 12 weeks after administration thereof, good responders, moderate responders and non responders were respectively 15, 11, and 7 according to the EULAR response criteria. Additionally, 7 patients entered remission (DAS28 (12w) < 2.6).

-1st Comparison between the Low group and the High group (ADAMTS4)-

In the 33 rheumatoid arthritis patients who received adalimumab, their expression level of ADAMTS4 mRNA (ADAMTS4/β-actin) before administration of adalimumab was quantified by real-time PCR method with a calibration curve method using the total cDNA from healthy human as a template for the control. Then, they were categorized into the Low group or the High group based on whether the expression level of ADAMTS4 mRNA (ADAMTS4/β-actin) was lower than $0.3 \times 10^{-4}$ or equal to or higher than $0.3 \times 10^{-4}$. The Low group, where the expression level thereof was lower than $0.3 \times 10^{-4}$, and the High group, where the expression level thereof was equal to or higher than $0.3 \times 10^{-4}$, were compared with each other in terms of DAS28 (12w). As a result, the DAS28 (12w) was found to be significantly low in the High group (see Fig. 1A).

-2nd Comparison between the Low group and the High group (ADAMTS5)-

In the 33 rheumatoid arthritis patients who received adalimumab, their expression level of ADAMTS5 mRNA (ADAMTS5/β-actin) before administration of adalimumab was quantified by real-time PCR method with a calibration curve method using the total cDNA from healthy human as a template for the control. Then, they were categorized into the Low group or the High group based on whether the expression level of ADAMTS5 mRNA (ADAMTS5/β-actin) was lower than $4.0 \times 10^{-4}$ or equal to or higher than $4.0 \times 10^{-4}$. The Low group, where the expression level thereof was lower than $4.0 \times 10^{-4}$, and the High group, where the expression level thereof was equal to or higher than $4.0 \times 10^{-4}$, were compared with each other in terms of DAS28 (12w). As a result, the DAS28 (12w) was found to be significantly low in the High group (see Fig. 1B).

--1st Comparison between the GR group and the NGR group (ADAMTS4)--

The Low group or the High group, into which the patients had been categorized based on the level of ADAMTS4 serving as an index, was further categorized into the GR group (good responders) or the NGR group (non responders + moderate responders) as shown in Table 1.

Table 1

| ADAMTS4 | GR | NGR | Total |
|---|---|---|---|
| High group | 6 | 1 | 7 |
| Low group | 9 | 17 | 26 |
| Total | 15 | 18 | 33 |
| $\chi^2$ p = 0.016 | | | |

Based on the categorization shown in Table 1, percentages of sensitivity, specificity, positive predictive value, and negative predictive value were calculated according to the following formulas. The results are shown in Table 2.

$$\text{Sensitivity (\%)} = A/(A + C) \times 100$$

$$\text{Specificity (\%)} = D/(D + B) \times 100$$

$$\text{Positive predictive value (PPV) (\%)} = A/(B + A) \times 100$$

$$\text{Negative predictive value (NPV) (\%)} = D/(D + C) \times 100$$

In the above formulas, A to D have the following meanings:
A: the number of patients categorized into the High group, where the expression level of ADAMTS4 mRNA (ADAMTS4/$\beta$-actin) was equal to or higher than $0.3 \times 10^{-4}$, and into the GR group;
B: the number of patients categorized into the High group, where the expression level of ADAMTS4 mRNA (ADAMTS4/$\beta$-actin) was equal to or higher than $0.3 \times 10^{-4}$, and into the NGR group;
C: the number of patients categorized into the Low group, where the expression level of ADAMTS4 mRNA (ADAMTS4/$\beta$-actin) was lower than $0.3 \times 10^{-4}$, and into the GR group; and
D: the number of patients categorized into the Low group, where the expression level of ADAMTS4 mRNA (ADAMTS4/$\beta$-actin) was lower than $0.3 \times 10^{-4}$, and into the NGR group.

Table 2

| Sensitivity | 40.0% |
|---|---|
| Specificity | 94.4% |
| PPV | **85.7**% |
| NPV | 65.4% |

--2nd Comparison between the GR group and the NGR group (ADAMTS5)--

The Low group or the High group, into which the patients had been categorized based on the level of ADAMTS5 serving as an index, was further categorized into the GR group (good responders) or the NGR group (non responders + moderate responders) as shown in Table 3.

Table 3

| ADAMTS5 | **GR** | **NGR** | **Total** |
|---|---|---|---|
| High group | 6 | 1 | 7 |
| Low group | 9 | 17 | 26 |

(continued)

| ADAMTS5 | GR | NGR | Total |
|---|---|---|---|
| **Total** | 15 | 18 | 33 |
| $\chi^2$ p = 0.016 | | | |

Based on the categorization shown in Table 3, percentages of sensitivity, specificity, positive predictive value, and negative predictive value were calculated according to the following formulas. The results are shown in Table 4.

$$\text{Sensitivity (\%)} = E/(E + G) \times 100$$

$$\text{Specificity (\%)} = H/(H + F) \times 100$$

$$\text{Positive predictive value (PPV) (\%)} = E/(F + E) \times 100$$

$$\text{Negative predictive value (NPV) (\%)} = H/(H + G) \times 100$$

In the above formulas, E to H have the following meanings:
E: the number of patients categorized into the High group, where the expression level of ADAMTS5 mRNA (ADAMTS5/$\beta$-actin) was equal to or higher than $4.0 \times 10^{-4}$, and into the GR group;
F: the number of patients categorized into the High group, where the expression level of ADAMTS5 mRNA (ADAMTS5/$\beta$-actin) was equal to or higher than $4.0 \times 10^{-4}$, and into the NGR group;
G: the number of patients categorized into the Low group, where the expression level of ADAMTS5 mRNA (ADAMTS5/$\beta$-actin) was lower than $4.0 \times 10^{-4}$, and into the GR group; and
H: the number of patients categorized into the Low group, where the expression level of ADAMTS5 mRNA (ADAMTS5/$\beta$-actin) was lower than $4.0 \times 10^{-4}$, and into the NGR group.

Table 4

| Sensitivity | 40.0% |
|---|---|
| Specificity | 94.4% |
| PPV | **85.7%** |
| NPV | 65.4% |

As shown in Tables 2 and 4, the positive predictive values (PPVs), predicting that patients whose expression level of ADAMTS4 mRNA (ADAMTS4/$\beta$-actin) or ADAMTS5 mRNA (ADAMTS5/$\beta$-actin) before administration of adalimumab was categorized into the High group would be categorized into the GR group, was found to be as high as 85.7%, indicating that the pharmacological efficacy of adalimumab against rheumatoid arthritis can be predicted based on an index that is the level of ADAMTS4 or ADAMTS5 before administration of adalimumab.

--1st Comparison between the remission (Re) group and non-remission (Nre) group (ADAMTS4)--

The Low group or the High group, into which the patients had been categorized based on the level of ADAMTS4 serving as an index, was further categorized into the Re (remission) group or the Nre (non-remission) group as shown in Table 5.

Table 5

| ADAMTS4 | **Remission** | **Non-remission** | **Total** |
|---|---|---|---|
| High group | 4 | 3 | 7 |

(continued)

| ADAMTS4 | Remission | Non-remission | Total |
|---|---|---|---|
| Low group | 3 | 23 | 26 |
| **Total** | 7 | 26 | 33 |
| $\chi^2$ p = 0.09 | | | |

Based on the categorization shown in Table 5, percentages of sensitivity, specificity, positive predictive value, and negative predictive value were calculated according to the following formulas. The results are shown in Table 6.

$$\text{Sensitivity (\%)} = \text{I}/(\text{I} + \text{K}) \times 100$$

$$\text{Specificity (\%)} = \text{L}/(\text{L} + \text{J}) \times 100$$

$$\text{Positive predictive value (PPV) (\%)} = \text{I}/(\text{J} + \text{I}) \times 100$$

$$\text{Negative predictive value (NPV) (\%)} = \text{L}/(\text{L} + \text{K}) \times 100$$

In the above formulas, I to L have the following meanings:

I: the number of patients categorized into the High group, where the expression level of ADAMTS4 mRNA (ADAMTS4/$\beta$-actin) was equal to or higher than $0.3 \times 10^{-4}$, and into the Re group;

J: the number of patients categorized into the High group, where the expression level of ADAMTS4 mRNA (ADAMTS4/$\beta$-actin) was equal to or higher than $0.3 \times 10^{-4}$, and into the Nre group;

K: the number of patients categorized into the Low group, where the expression level of ADAMTS4 mRNA (ADAMTS4/$\beta$-actin) was lower than $0.3 \times 10^{-4}$, and into the Re group; and

L: the number of patients categorized into the Low group, where the expression level of ADAMTS4 mRNA (ADAMTS4/$\beta$-actin) was lower than $0.3 \times 10^{-4}$, and into the Nre group.

Table 6

| Sensitivity | 57.1% |
|---|---|
| Specificity | 88.5% |
| PPV | **57.1%** |
| NPV | 88.5% |

--2nd Comparison between the remission (Re) group and non-remission (Nre) group (ADAMTS5)--

The Low group or the High group, into which the patients had been categorized based on the level of ADAMTS5 serving as an index, was further categorized into the Re (remission) group or the Nre (non-remission) group as shown in Table 7.

Table 7

| ADAMTS5 | Remission | Non-remission | Total |
|---|---|---|---|
| High group | 5 | 2 | 7 |
| Low group | 2 | 24 | 26 |
| **Total** | 7 | 26 | 33 |
| $\chi^2$ p = 0.0003 | | | |

Based on the categorization shown in Table 7, percentages of sensitivity, specificity, positive predictive value, and negative predictive value were calculated according to the following formulas. The results are shown in Table 8.

$$\text{Sensitivity (\%)} = M/(M + O) \times 100$$

$$\text{Specificity (\%)} = P/(P + N) \times 100$$

$$\text{Positive predictive value (PPV) (\%)} = M/(N + M) \times 100$$

$$\text{Negative predictive value (NPV) (\%)} = P/(P + O) \times 100$$

In the above formulas, M to P have the following meanings:

M: the number of patients categorized into the High group, where the expression level of ADAMTS5 mRNA (ADAMTS5/β-actin) was equal to or higher than $4.0 \times 10^{-4}$, and into the Re group;

N: the number of patients categorized into the High group, where the expression level of ADAMTS5 mRNA (ADAMTS5/β-actin) was equal to or higher than $4.0 \times 10^{-4}$, and into the Nre group;

O: the number of patients categorized into the Low group, where the expression level of ADAMTS5 mRNA (ADAMTS5/β-actin) was lower than $4.0 \times 10^{-4}$, and into the Re group; and

P: the number of patients categorized into the Low group, where the expression level of ADAMTS5 mRNA (ADAMTS5/β-actin) was lower than $4.0 \times 10^{-4}$, and into the Nre group.

Table 8

| | |
|---|---|
| Sensitivity | 71.4% |
| Specificity | 92.3% |
| PPV | **71.4%** |
| NPV | 92.3% |

As shown in Tables 6 and 8, the positive predictive values (PPVs), predicting that patients whose expression level of ADAMTS4 mRNA (ADAMTS4/β-actin) or ADAMTS5 mRNA (ADAMTS5/β-actin) before administration of adalimumab was categorized into the High group would be categorized into the Re group, were found to be as high as 57.1% in the case of ADAMTS4 and 71.4% in the case of ADAMTS5.

Also, the negative predictive value (NPV), predicting that patients whose expression level of ADAMTS4 mRNA (ADAMTS4/β-actin) or ADAMTS5 mRNA (ADAMTS5/β-actin) before administration of adalimumab was categorized into the Low group would be categorized into the Nre group, were found to be considerably high; i.e., 88.5% in the case of ADAMTS4 and 92.3% in the case of ADAMTS5.

These results indicate that whether or not patients enter remission can be predicted based on an index that is the level of ADAMTS4 or ADAMTS5 before administration of adalimumab.

--Prediction 1: DAS28 after administration of adalimumab (ADAMTS4)--

Fig. 2A shows correlation between the expression level of ADAMTS4 mRNA (ADAMTS4/β-actin) in peripheral blood before administration of adalimumab and the change of DAS28 (12w) at 12 weeks after administration of adalimumab.

As shown in Fig. 2A, the expression level of ADAMTS4 mRNA (ADAMTS4/β-actin) before administration of adalimumab was found to be negatively correlated (r = -0.370) with the change of DAS28 (12w) at 12 weeks after administration of adalimumab.

That is, these results indicate that the more the level of ADAMTS4 in a sample derived from a subject before administration of adalimumab, the more efficacious a human anti-TNFα antibody drug against rheumatoid arthritis, and that the efficacy of adalimumab at 12 weeks after administration of a human anti-TNFα antibody drug can be quantified and predicted based on an index that is the level of ADAMTS4 in a sample derived from a subject before administration of adalimumab.

--Prediction 2: DAS28 after administration of adalimumab (ADAMTS5)--

Fig. 2B shows correlation between the expression level of ADAMTS5 mRNA (ADAMTS5/β-actin) in peripheral blood before administration of adalimumab and the change of DAS28 (12w) at 12 weeks after administration of adalimumab.

As shown in Fig. 2B, the expression level of ADAMTS5 mRNA (ADAMTS5/β-actin) before administration of adalimumab was found to be negatively correlated (r = -0.476) with the change of DAS28 (12w) at 12 weeks after administration of adalimumab.

That is, these results indicate that the more the level of ADAMTS5 in a sample derived from a subject before administration of adalimumab, the more efficacious a human anti-TNFα antibody drug against rheumatoid arthritis, and that the efficacy of adalimumab at 12 weeks after administration of a human anti-TNFα antibody drug can be quantified and predicted based on an index that is the level of ADAMTS5 in a sample derived from a subject before administration of adalimumab.

Industrial Applicability

The method of the present invention for predicting pharmacological efficacy of a human anti-TNFα antibody drug against rheumatoid arthritis can predict the efficacy of a human anti-TNFα antibody drug against rheumatoid arthritis with high reliability and in a simple manner as well as can predict the activity of rheumatoid arthritis after administration of the anti-TNFα antibody drug based on an index that is the level of at least one of ADAMTS4 and ADAMTS5 at a point of time before administration of the human anti-TNFα antibody drug, resulting in that the human anti-TNFα antibody drug can be administered to patients on which the human anti-TNFα antibody drug reliably takes effects with preventing unwanted side effects. Thus, the method of the present method can be suitably used for medical diagnosis and treatments.

The non-claimed apparatus for predicting pharmacological efficacy of a human anti-TNFα antibody drug against rheumatoid arthritis can predict the efficacy of a human anti-TNFα antibody drug against rheumatoid arthritis with high reliability and in a simple manner as well as can predict the activity of rheumatoid arthritis after administration of the human anti-TNFα antibody drug based on an index that is the level of at least one of ADAMTS4 and ADAMTS5 at a point of time before administration of the human anti-TNFα antibody drug, resulting in that the human anti-TNFα antibody drug can be administered to patients on which the human anti-TNFα antibody drug reliably takes effects with preventing unwanted side effects. Thus, the (non-claimed) apparatus can be suitably used for medical diagnosis and treatments.

**Claims**

1. A method for predicting pharmacological efficacy of a human anti-TNFα antibody drug against rheumatoid arthritis, the method comprising:

   measuring a level of at least one of ADAMTS4 and ADAMTS5 in a sample derived from a subject, and determining whether or not the human anti-TNFα antibody drug is efficacious against rheumatoid arthritis of the subject, based on the level of the at least one of ADAMTS4 and ADAMTS5 serving as an index, wherein the level of the at least one of ADAMTS4 and ADAMTS5 in the sample derived from the subject is an expression level of at least one of ADAMTS4 mRNA and ADAMTS5 mRNA, and wherein the determining is determining that the human anti-TNFα antibody drug is efficacious against rheumatoid arthritis of the subject, when the expression level of ADAMTS4 mRNA is equal to or higher than $0.3 \times 10^{-4}$ as a relative value (ADAMTS4/β-actin) to a level of mRNA of β-actin which is an endogenous control, or when the expression level of ADAMTS5 mRNA is equal to or higher than $4.0 \times 10^{-4}$ as a relative value (ADAMTS5/β-actin) to the level of the mRNA of the β-actin which is the endogenous control, or when the expression level of ADAMTS4 mRNA is equal to or higher than $0.3 \times 10^{-4}$ as the relative value (ADAMTS4/β-actin) and the expression level of ADAMTS5 mRNA is equal to or higher than $4.0 \times 10^{-4}$ as the relative value (ADAMTS5/β-actin).

2. The method according to claim 1, wherein the human anti-TNFα antibody drug is adalimumab (ADA).

3. The method according to claim 1 or 2, wherein the expression level of the at least one of ADAMTS4 mRNA and ADAMTS5 mRNA is measured by real-time PCR method.

4. The method according to any one of claims 1 to 3, wherein the at least one of ADAMTS4 and ADAMTS5 is ADAMTS5.

**Patentansprüche**

1. Ein Verfahren zur Vorhersage der pharmakologischen Wirksamkeit eines humanen anti-TNFα-Antikörper-Wirkstoffs gegen rheumatoide Arthritis, wobei das Verfahren umfasst:

Messen der Konzentration von mindestens einem von ADAMTS4 und ADAMTS5 in einer Probe, die einem Subjekt entnommen worden ist; und
Bestimmen, ob der humane anti-TNFα-Antikörper-Wirkstoff wirksam ist gegen rheumatoide Arthritis des Subjekts oder nicht, auf Basis der Konzentration von mindestens einem von ADAMTS4 und ADAMTS5, die als Indexparameter dienen,
wobei die Konzentration von mindestens einem von ADAMTS4 und ADAMTS5 in der Probe, die einem Subjekt entnommen worden ist, das Expressionsniveau von mindestens einem von ADAMTS4 mRNA und ADAMTS5 mRNA ist, und
wobei das Bestimmen das Bestimmen ist, dass der humane anti-TNFα-Antikörper-Wirkstoff, der gegen rheumatoide Arthritis des Subjekts wirksam ist, wenn das Expressionsniveau der ADAMTS4 mRNA gleich oder höher als $0,3 \times 10^{-4}$ als relativer Wert (ADAMTS4/β-Actin) gegenüber einem Niveau der mRNA von β-Actin, welche eine endogene Kontrolle ist, ist, oder wenn das Expressionsniveau von ADAMTS5 mRNA gleich oder höher als $4.0 \times 10^{-4}$ als relativer Wert (ADAMTS5/β-Actin) gegenüber dem Niveau der mRNA von β-Actin ist, welche eine endogene Kontrolle ist, oder wenn das Expressionsniveau von ADAMTS4 mRNA gleich oder höher ist als $0.3 \times 10^{-4}$ als relativer Wert (ADAMTS4/β-Actin) und das Expressionsniveau von ADAMTS5 mRNA gleich oder höher als $4.0 \times 10^{-4}$ als relativer Wert (ADAMTS5/β-Actin) ist.

2. Verfahren nach Anspruch 1, wobei der humane anti-TNFα-Antikörper-Wirkstoff Adalimumab (ADA) ist.

3. Verfahren nach Anspruch 1 oder 2, wobei das Expressionsniveau von dem mindestens einen von ADAMTS4 mRNA und ADAMTS5 mRNA gemessen wird durch ein "real-time" PCR-Verfahren.

4. Verfahren nach einem der Ansprüche 1 bis 3, wobei das mindestens eine von ADAMTS4 und ADAMTS5 ADAMTS5 ist.

**Revendications**

1. Procédé de prédiction de l'efficacité pharmacologique d'un médicament à base d'un anticorps anti-TNFα humain sur la polyarthrite rhumatoïde, le procédé comprenant :

la mesure d'un taux d'au moins un parmi ADAMTS4 et ADAMTS5 dans un échantillon provenant d'un sujet, et le fait de déterminer si le médicament à base d'un anticorps anti-TNFα humain est efficace ou non contre la polyarthrite rhumatoïde du sujet, sur la base du taux du au moins un parmi ADAMTS4 et ADAMTS5 servant d'indice,
dans lequel le taux du au moins un parmi ADAMTS4 et ADAMTS5 dans l'échantillon provenant du sujet est un taux d'expression d'au moins un parmi l'ARNm d'ADAMTS4 et l'ARNm d'ADAMTS5, et
dans lequel la détermination détermine que le médicament à base d'un anticorps anti-TNFα humain est efficace contre la polyarthrite rhumatoïde du sujet quand le taux d'expression de l'ARNm d'ADAMTS4 est supérieur ou égal à $0,3 \times 10^{-4}$ en tant que valeur relative (ADAMTS4/β-actine) par rapport à un taux d'ARNm de β-actine qui est un témoin endogène, ou quand le taux d'expression de l'ARNm d'ADAMTS5 est supérieur ou égal à $4,0 \times 10^{-4}$ en tant que valeur relative (ADAMTS5/β-actine) par rapport au taux d'ARNm de la β-actine qui est un témoin endogène, ou quand le taux d'expression de l'ARNm d'ADAMTS4 est supérieur ou égal à $0,3 \times 10^{-4}$ en tant que valeur relative (ADAMTS4/β-actine) et le taux d'expression de l'ARNm d'ADAMTS5 est supérieur ou égal à $4,0 \times 10^{-4}$ en tant que valeur relative (ADAMTS5/β-actine).

2. Procédé selon la revendication 1, dans lequel le médicament à base d'un anticorps anti-TNFα humain est l'adalimumab (ADA).

3. Procédé selon la revendication 1 ou 2, dans lequel le taux d'expression du au moins un parmi l'ARNm d'ADAMTS4 et l'ARNm d'ADAMTS5 est mesuré par un procédé PCR en temps réel.

4. Procédé selon l'une quelconque des revendications 1 à 3, dans lequel le au moins un parmi ADAMTS4 et ADAMTS5

est ADAMTS5.

# FIG. 1A

p=0.0484

High group
(n=7)

Low group
(n=26)

ADAMTS4 before administration

# FIG. 1B

p=0.0092

ADAMTS5 before administration

# FIG. 2A

ADAMTS4/β-actin before administration

# FIG. 2B

ADAMTS5/β-actin before administration

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2343372 A

**Non-patent literature cited in the description**

- **BADOT V et al.** *Arthritis Research Therapy,* 2009, vol. 11, R57
- The 54rd annual general assembly and scientific meeting. **KENSEI TSUZAKA et al.** Japan College of Rheumatology the 18th International Rheumatology Symposium. Japan College of Rheumatology, 01 March 2009, vol. 53rd-18, 274
- **FRANSEN J et al.** *Clin Exp Rheumatol,* 2005, vol. 23 (39), S93-S99
- *Division of Rheumatology, Department of Internal Medicine, Saitama Medical Center,* June 2008